Europäisches Patentamt

European Patent Office

Office européen des brevets

(11) Publication number: **0 499 368 A1**

# EUROPEAN PATENT APPLICATION

(21) Application number: **92300620.9**

(22) Date of filing: **24.01.92**

(51) Int. Cl.⁵: **A61M 5/158**

(30) Priority: **15.02.91 GB 9103284**

(43) Date of publication of application:
**19.08.92 Bulletin 92/34**

(84) Designated Contracting States:
**DE DK ES FR IT SE**

(71) Applicant: **Smiths Industries Public Limited Company**
**765, Finchley Road**
**London, NW11 8DS(GB)**

(72) Inventor: **Summers, Raymond Jeffrey**
**Cornerstone, 9 The Brush, Cashes Green**
**Stroud, Gloucestershire GL5 4SH(GB)**

(74) Representative: **Flint, Jonathan McNeill**
**SMITHS INDUSTRIES PUBLIC LIMITED**
**COMPANY 765 Finchley Road**
**London NW11 8DS(GB)**

(54) Intravenous needle assemblies.

(57) An intravenous needle assembly has a transparent plastic hub 2 the patient end of which is moulded on the end of a hollow needle 1 and the machine end of which is bonded to flexible tubing 3. The tubing 3 extends in the machine end of the bore 23 through the hub 2 in abutment with an internal shoulder 24. The patient end of the bore 23 tapers from a diameter equal to the internal diameter of the tubing 3 to a diameter equal to the internal diameter of the needle 1 so that there is a smooth flow from the tubing into the needle. A winged handle 30 is clipped to the hub 2.

*Fig. 1.*

This invention relates to intravenous needle assemblies of the kind comprising a hollow needle having a patient end which in use is inserted in a blood vessel of a patient, a hub mounted on the machine end of the needle and having a bore therethrough in communication with the interior of the needle and tubing connected with the hub.

Intravenous needle assemblies of the general type with which the present invention is concerned are known in the prior art. However, the devices presently available show certain deficiencies and disadvantages. The prior art assemblies couple the tubing to the needle by means of a spigot which is inserted into the end of the tubing. Inherent in this arrangement is that a sudden change in the internal diameter of the system can occur between the tubing and the spigot. The fluid flow will be impeded at this junction, causing an increase of pressure in this region and a reduction in flow rate through the assembly. This problem is more acute if the leading edge of the spigot is not entirely smooth because of, for example, residual flash from the manufacturing process.

In techniques such as diafiltration, dialysis and plasmaphoresis, a fast flow rate is desired as this reduces the time that the patient has to undergo treatment. In present systems, the flow rate can be limited by the back-pressure set up in the joint between the tubing and spigot. It would be possible to increase the flow rate by increasing the needle diameter, however, this causes a larger puncture hole in the blood vessel and is more traumatic.

It is an object of the present invention to provide an improved form of intravenous needle assembly which can afford a higher flow rate, for a given cannula diameter.

According to the present invention there is provided an intravenous needle assembly of the above-specified kind, characterised in that the bore through the hub has a first patient end portion and a second machine end portion, that the second portion has a diameter substantially equal to the external diameter of the tubing, that the bore has a shoulder between the first and second portions, that the first portion has a diameter at the shoulder substantially equal to the internal diameter of the tubing, and that the tubing abuts the shoulder so that there is a smooth transition for fluid flow between the tubing and the first portion of the bore.

The first portion of the bore preferably tapers to a smaller diameter adjacent the machine end of the needle. The hub may be of a plastics material and be moulded to the needle. The hub is preferably of a transparent material. The assembly may include a winged handle mounted on the hub. The machine end of the hub preferably projects from the handle to provide a stable platform for gripping between the finger and thumb. The handle may be located on the hub between two projections on the hub. The underside of the handle and hub preferably provides a substantially smooth surface to contact the skin of the patient.

An intravenous needle assembly in accordance with the present invention, will now be described, by way of example, with reference to the accompanying drawings, in which:

Figure 1    is a plan sectional side elevation view of the assembly;

Figure 2    is a plan view of the assembly in conjunction with the preferred angled wing handle;

Figure 3    is a side elevation view of the assembly shown in Figure 2 with the handle folded up; and

Figure 4    is a graph illustrating the performance of the assembly compared with conventional assemblies.

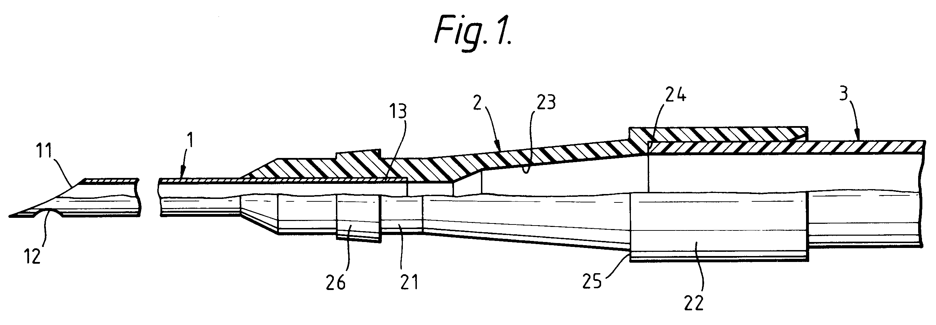

With reference to Figure 1, the intravenous needle assembly comprises a needle member 1, a hub member 2 and tubing 3.

The needle member 1 comprises a hollow stainless steel needle having a bevelled, pointed tip 11 with a vent opening 12 at the patient end. The machine end 13 of the needle 1 is moulded, or otherwise fixedly retained, in the patient end of the hub member 2.

The hub member 2 is hollow, of generally cylindrical shape and circular section, and is moulded from a transparent, rigid plastics material, such as polycarbonate. The hub member 2 comprises a first, patient end portion 21, within which the machine end of the needle 1 is retained, and a second, machine end portion 22, within which the tubing 3 is retained. Internally, the hub 2 has a bore 23 which tapers along the length of the patient end portion 21. The patient end of the bore 23 makes a smooth transition with the bore through the machine end 13 of the needle 1, being 1.49mm in diameter for a 16 gauge needle. The machine end of the bore 23 is of greater diameter, typically 3.5mm, and terminates at an annular shoulder 24 which forms a step with the bore through the patient end portion 22. The bore of the machine end portion 22 has a constant diameter of 5mm along its length, so that this part of the bore is of greater diameter than the part of the bore through the largest patient end portion 21. The diameter of the bore 23 in the machine portion 22 is equal to the external diameter of the tubing 3, whereas the diameter of the bore in the patient end portion 21 at the shoulder 24 is equal to the internal diameter of the tubing 3.

The tubing 3 is of a conventional, soft, flexible transparent plastics such as PVC. At its patient end, the tubing 3 is inserted within the machine end 22 of the hub 2 by a distance of 7.5mm with the end of the tubing abutting the shoulder 24. The tubing 3 is bonded into the hub by means of a solvent applied to its external surface. At its machine end, the tubing 3 is provided with a conventional coupling, not shown.

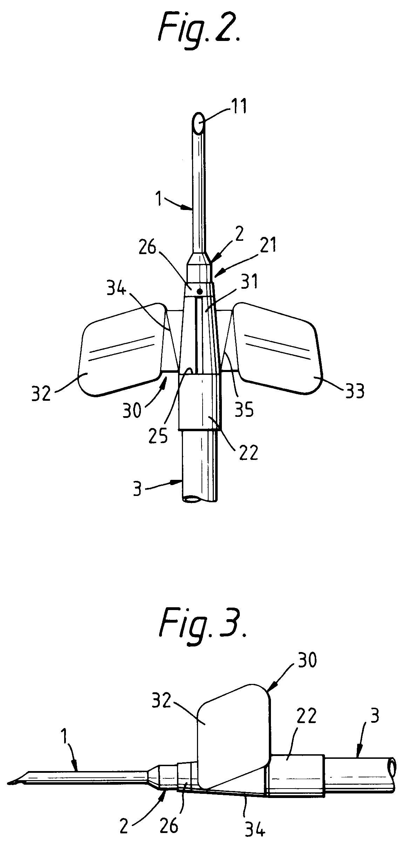

With reference to Figures 2 and 3, the assembly is generally used in conjunction with a winged handle 30. The handle 30 is described in detail in GB 2242361A. The handle 30 has a central portion 31 which clips about the patient end portion 21 of the hub 2 and is located between two projections formed by an external shoulder 25 at the transition between two projections formed by the patient and machine end portions, at one end, and an annular flange 26 close to the patient end of the hub, at its other end. The handle 30 has two wings 32 and 33 which can be folded up about respective fold lines 34 and 35 to the position shown in Figure 3. The thickness of the underside of the central portion 31 is such that its external surface lies level with the exterior of the machine end 22 of the hub, so that the underside of the assembly provides a substantially smooth surface to contact the skin of the patient. The machine end 22 of the hub 2 projects from the machine end of the handle 30 and, because it is of relatively large diameter (that is, it is larger than the tubing 3), it helps provide, in conjunction with the handle, a stable platform for the user's fingers when the wings 32 and 33 are gripped between the index finger and thumb. This enables a greater accuracy during insertion of the needle.

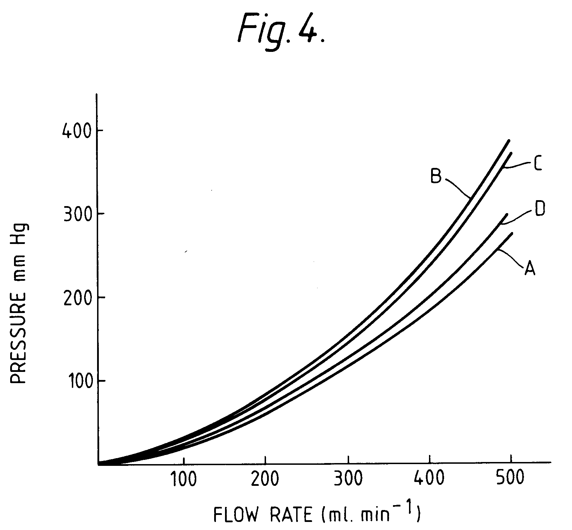

With reference to Figure 4, the flow rate of the assembly is shown by the curve A, in comparison with those of three other conventional assemblies B, C and D employing the same gauge needle. It can be seen that, for any given pressure, the flow rate of the present invention is markedly greater. This is achieved because of the smooth flow characteristics through the assembly and, in particular, because of the smooth transition from the bore of the tubing 3 to the bore through the hub 2, as a result of inserting the tubing in the hub, rather than inserting a spigot in the tubing. In previous assemblies, attempts have been made to smooth the transition with the end of the spigot inserted in the tubing, by reducing the wall thickness of the spigot at its end. This, however, causes difficulties with moulding and can result in ragged edges to the spigot which themselves increase turbulence and reduce flow rate.

The increased flow rate achieved by the present invention enables shorter treatment times, or enables the same flow rate to be achieved by using a smaller diameter needle, thereby reducing the risk of damage to blood vessels.

## Claims

1. An intravenous needle assembly of the kind comprising a hollow needle having a patient end which in use is inserted in a blood vessel of a patient, a hub mounted on the machine end of the needle and having a bore therethough in communication with the interior of the needle, and tubing connected with the hub, characterised in that the bore (23) through the hub (2) has a first patient end portion and a second machine end portion, that the second portion has a diameter substantially equal to the exterior diameter of the tubing (3), that the bore (23) has a shoulder (24) between the first and second portions, that the first portion has a diameter at the shoulder (24) substantially equal to the internal diameter of the tubing (3), and that the tubing (3) abuts the shoulder (24) so that there is a smooth transition for fluid flow between the tubing (3) and the first portion of the bore (23).

2. An assembly according to Claim 1, characterised in that the first portion of the bore (23) tapers to a smaller diameter adjacent the machine end of the needle (1).

3. An assembly according to Claim 1 or 2, characterised in that the hub (2) is of a plastics material and is moulded to the needle (1).

4. An assembly according to any one of the preceding claims, characterised in that the hub (2) is of a transparent material.

5. An assembly according to any one of the preceding claims, characterised in that the assembly includes a winged handle (30) mounted on the hub (2).

6. An assembly according to Claim 5, characterised in that the machine end of the hub (2) projects from the handle (30) to provide a stable platform for gripping between the finger and thumb.

7. An assembly according to Claim 5 or 6, characterised in that the handle (30) is located on the hub (2) between two projections (25 and 26) on the hub.

8. An assembly according to any one of Claims 5 to 7, characterised in that the underside of the handle (30) and hub (2) provides a substantially smooth surface to contact the skin of the patient.

Fig. 1.

# Fig.2.

# Fig.3.

Fig. 4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 142 223 (WARNER-LAMBERT CO.) <br> * claim 1; figures 1-3 * <br> --- | 1,3-5,8 | A61M5/158 |
| X | FR-A-2 371 630 (GAMBRO DIALYSATOREN GMBH.) <br><br> * page 2, line 32 - page 3, line 35; figures 1-3 * <br><br> --- | 1-2,5-6, 8 | |
| Y | WO-A-9 003 195 (SÜDDEUTSCHE FEINMECHANIK GMBH) <br> * page 7, line 28 - page 8, line 38; figures 1,5-10 * <br> --- | 1-8 | |
| Y | EP-A-0 356 774 (EDWARD WECK, INC.) <br> * column 7, line 23 - line 48; figure 4 * <br> --- | 1-8 | |
| A | US-A-4 585 444 (HARRIS) <br> * abstract; figures * | 1-8 | |
| | ----- | | **TECHNICAL FIELDS SEARCHED (Int. Cl.5)** <br><br> A61M |

The present search report has been drawn up for all claims

| Place of search | Date of completion of the search | Examiner |
|---|---|---|
| THE HAGUE | 15 JUNE 1992 | MIR Y GUILLEN V. |

EPO FORM 1503 03.82 (P0401)